# EUROPEAN PATENT SPECIFICATION

(11) **EP 0 350 004 B1**
(45) Date of publication and mention of the grant of the patent: **11.05.1994**
(21) Application number: 89112269.9
(22) Date of filing: 05.07.1989
(51) Int. Cl.: G01N 33/50, G01N 33/579, B01D 15/00

(54) **Method for determination of pyrogen content**
Methode zur Bestimmung des Pyrogengehaltes
Procédé pour la détermination de la teneur en pyrogènes

(30) Priority: 05.07.1988 JP 168063/88; 28.10.1988 JP 274249/88
(43) Date of publication of application: 10.01.1990
(73) Proprietor: TANABE SEIYAKU CO., LTD., Chuo-ku, Osaka-shi, Osaka-fu (JP)
(72) Inventor: Tosa, Tetsuya, Kyoto-shi Kyoto-fu (JP); Sato, Tadashi, Takatsuki-shi Osaka-fu (JP); Watanabe, Taizo, Nagaokakyo-shi Kyoto-fu (JP); Minobe, Satoshi, Otsu-shi Shiga-ken (JP)
(74) Representative: Hansen, Bernd, Dr. Dipl.-Chem.

(56) References cited:
- GB-A- 2 092 470
- US-A- 3 944 391
- US-A- 4 276 050
- JOURNAL OF CHROMATOGRAPHY, vol. 476, 1989; pp. 175-185#

## Description

The present invention relates to a method for determination of a pyrogen content. More particularly, it relates to a method for determination of a pyrogen content which is applicable to even a specimen which has hitherto been difficult to determine because of the presence of determination interfering substances and the like.

Pyrogens are pyrogenetic substances which abnormally raise the body temperature of a homothermic animal in a very small amount. When a pyrogen is introduced into the human body, for example, by intravenous injection of a medicine contaminated with it, apart from the main activity of the medicine, the pyrogen causes severe fever. It is said that, when this action of pyrogen becomes serious, it causes severe fever accompanied with chill and shudder and, occasionally, death from a shock. Many substances such as bacterial substances, inflammatory substances, plant polysaccharides, blood group substances and the like have been known as pyrogens. Among them, bacterial substances have the most important influence on fever and are called as bacterial toxins. In general, bacterial toxins are classified into exotoxins and endotoxins. Particularly, it is said that an endotoxin acting as so-called O-antigen the main component of which is a cell wall lipopolysaccharide (hereinafter referred to as LPS) of a gram negative bacterium has the strongest pyrogenicity.

Accordingly, it is very important to detect or determine a pyrogen content, for example, to prevent contamination of a pyrogen in the production of medicines.

As a method for detecting or determining a pyrogen content, for example, there has been hitherto known fever test using a rabbit, or Limulus test using a blood cell extract of Limulus polyphenus. Particularly, Limulus test has been often used from viewpoints of sensitivity, simplicity, quantitative properties and the like.

However, Limulus test is liable to be interfered or activated by various substances present during determination and, therefore, complicated pre-treatment is required or, sometimes, it becomes difficult to determine the pyrogen content depending upon a specimen. Further, there are some substances other than pyrogens which are positive in Limulus test and they interfere precise determination of the pyrogen content. In order to solve this problem, it has been proposed to use highly purified reagents. However, such reagents are very expensive. Further, it is considered to be difficult to determine a very small amount of pyrogens contained in a substance having a low solubility.

Recently, in order to detect an endotoxin which is one of pyrogens, there has been proposed a method which comprises bringing a pyrogen-free activated charcoal obtained by treatment with an acid into contact with a specimen and reacting the activated charcoal with Limulus lysate to carry out Limulus test easily and rapidly (Japanese Patent Laid Open Publication No. 152425/1981). However, this method is yet unsatisfactory from viewpoints of specificity, sensitivity and quantitative properties for pyrogens. Further, U.S. Patents No. 3,944,391 and 4,491,660 disclose that a certain polymer which can adsorb an endotoxin can be used for concentrating and detecting the endotoxin. However, this polymer is yet unsatisfactory from viewpoints of specificity, sensitivity and quantitative properties for pyrogens, too.

The present inventors have already found that pyrogens are specifically adsorbed by an adsorbent comprising a nitrogen-containing heterocyclic compound bonded to a water-insoluble carrier directly or through a spacer and has filed a patent application GB-A-2092470.

Then, the present inventors have further studied and found that, by using some of such an adsorbent, a pyrogen content can be readily carried out by Limulus test in high specificity and sensitivity even in the presence of various interfering substances or other Limulus test positive substances.

The main object of the present invention is to provide an improved method for determination of a pyrogen content by Limulus test.

This object as well as other objects and advantages of the present invention will become apparent to those skilled in the art from the following description with reference to the accompanying drawings.

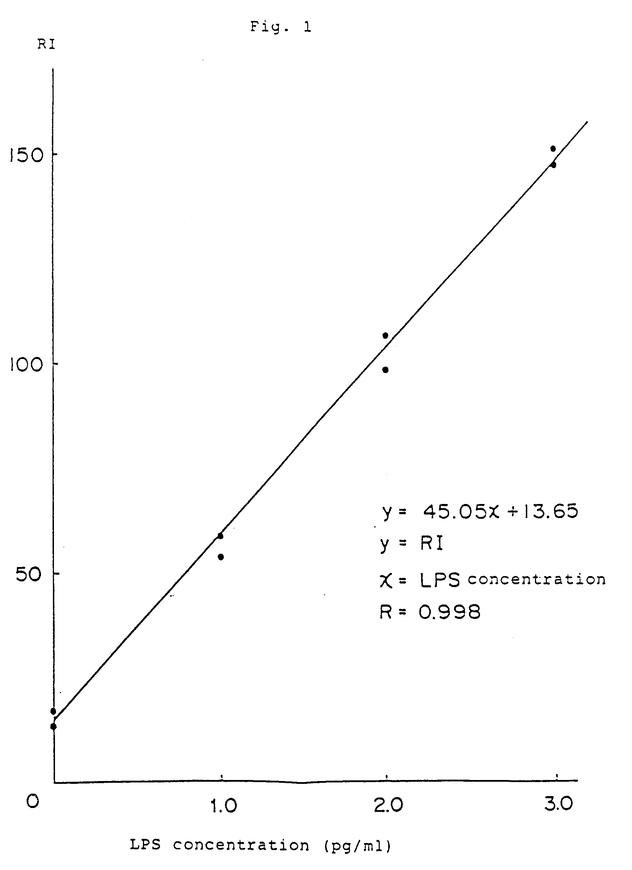

Figs. 1 to 3 are examples of calibration curves used in the determination of the present invention, respectively.

According to the present invention, there is provided a method for determination of a pyrogen content which comprises bringing a specimen into contact with an adsorbent composed of a nitrogen-containing heterocyclic compound bonded to a water-insoluble carrier through a spacer, and without eluting the pyrogen adsorbed on the adsorbent, determining the pyrogen content of the adsorbed pyrogen by means of Limulus method; wherein said nitrogen-containing heterocyclic compound is compound (I) of formula

R-A-X (I)

wherein R is a nitrogen-containing heterocyclic group having an imidazole nucleus or a purine nucleus;
A is a single bond or an alkylene group;
X is a functional group;
and the alkylene group may be substituted with a carboxyl group; and said spacer being a compound of the formula:

NH₂(CH₂)ₙ NH₂,

NH₂(CH₂)ₙ COOH

NH₂(CH₂)ₙ OH

or

HOOC(CH₂)ₙ COOH

wherein n is an integer of 1 to 12.

In the above-mentioned adsorbent to be used in the present invention, examples of the nitrogen-containing heterocyclic compound of the formula [I] include those wherein R is a nitrogen-containing heterocyclic group having an imidazole nucleus, or a, purine nucleus. A is a single bond or an alkylene group having 1 to 12 carbon atoms such as methylene, ethylene, propylene, butylene, hexylene, octylene, decamethylene or dodecamethylene and X is a functional group such as amino, hydroxy or carboxyl. The the alkylene group of A may be optionally substituted with a carboxyl group. Preferred examples of the nitrogen-containing heterocyclic compounds are those of the formula [I] wherein R is a nitrogen-containing heterocyclic group having imidazole nucleus or purine nucleus, A is a single bond, ethylene or ethylene substituted with carboxyl, and X is amino.

As the water-insoluble carrier, any water-insoluble carrier which can be bonded to the nitrogen-containing heterocyclic compound of the general formula [I] through a spacer can be used in the present invention. Representative examples of the water-insoluble carrier include those having hydroxy, amino, carboxyl or a halogen atom. Preferred examples of the water-insoluble carrier having hydroxy are polysaccharides (e.g. cellulose, agarose, cross-linked dextran, etc.), hydroxyalkylated polystyrene resins (e.g. hydroxyalkylated styrene-divinylbenzene copolymer, etc.), polyvinyl alcohol or the like. Examples of the water-insoluble carrier having amino group are aminoalkylated polysaccharides (e.g. aminoalkylated celluloses such as aminoethylcellulose or aminohexylcellulose, aminoalkylated agarose such as aminohexylagarose, a p-aminobenzylated polysaccharides (e.g. p-aminobenzylcellulose, p-aminobenzylagarose, etc.), chitosan, aminoalkylated polystyrene resins (e.g. aminoalkylated styrene-divinylbenzene copolymer, etc.), polyacrylamides, aminoalkylated polyacrylamides (e.g. aminoethylpolyacrylamide, etc.), aminoalkylated porous glass (e.g. aminopropyl porous glass, etc.) and the like. Examples of the water-insoluble carrier having carboxyl group are carboxyalkylated polysaccharides (e.g. carboxyalkylated agarose such as carboxyhexylagarose or carboxypentylagarose, carboxyalkylated celluloses such as carboxymethylcellulose, carboxyalkylated cross-linked dextran such as carboxymethyl-crosslinked dextran, etc.), carboxylic acid resins [e.g. acrylic acid-divinylbenzene copolymer, etc.) and the like. Examples of the water-insoluble carrier having a halogen atom are halogenoalkylpolystyrene resins (e.g. chloromethylated styrene-divinylbenzene copolymer, etc.) and the like.

Preferred examples of the adsorbent used in the present invention are those obtained by bonding the nitrogen-containing heterocyclic compound [I] such as histidine, histamine or adenine to the water-insoluble polysaccharide through alkylenediamine as the spacer by using, at least, either one of a monoepoxide (e.g. epichlorohydrin, etc.) and an aliphatic dialdehyde (e.g. glutaraldehyde, etc.).

Further preferred examples of the adsorbent used in the present invention are those obtained by bonding histidine, histamine or adenine to a water-insoluble polysaccharide being selected from cellulose and agarose through an alkylenediamine by epichlorohydrin.

The adsorbent used in the present invention is characterized in that it specifically adsorbs only pyrogens. Thus, the method of the present invention can be carried out by bringing the adsorbent into contact with a specimen to adsorb a pyrogen in the specimen, washing the specimen to remove substances other than the pyrogen without eluting the adsorbed pyrogen, subjecting it to a conventional Limulus test to determine the pyrogen content.

In the method of the present invention, the specimen to be examined should be used in a dissolved state, for example, as a water-containing solution or an aqueous solution. An aqueous solution is preferred. The adsorbent used in the present invention does not adsorb interfering substances, for example, amino acids (e.g. L-cysteine, etc.), antibiotics (e.g. penicillin G, streptomycine, etc.), protein denaturants (e.g. urea, etc.), serine protease inhibitors (e.g. diisopropylfluorophosphoric acid, etc.), saccharides (e.g. glucose, etc.), sugar alcohols, sodium chloride, Ringer's solution and the like. The adsorbent does not adsorb Limulus test positive substances other than pyrogens, for example, polysaccharides such as dextran, (1→3)- β-D-glucan and the like, either. Therefore, the method of the present invention can also be applied to a specimen containing these substances which have hitherto been difficult to determine the pyrogen content.

The adsorption operation of the pyrogen can be carried out by bringing the specimen into contact with the adsorbent, for example, by mixing and stirring a specimen with an adsorbent in a container or by passing the specimen through a column packed with the adsorbent. The conditions of the adsorption operation can be appropriately selected according to a particular specimen. Usually, the pyrogen can be specifically and efficiently adsorbed by using 3 to 100 mg, preferably 7 to 40 mg of the adsorbent per 1 ml of the specimen and carrying out the adsorption at a temperature of 4 to 40°C, pH of 4 to 8, preferably, 6 to 7 and an ionic strength of not more than 0.1, preferably 0 to 0.05. Under such conditions, by carrying out the adsorption operation for about 30 minutes or longer, the pyrogen in the specimen is almost completely adsorbed on the adsorbent and the pyrogen is condensed on the adsorbent. Further, re-adsorption can also be carried out after completion of adsorption and thereby the sensitivity of determination can be raised.

The adsorbent which has adsorbed the pyrogen is separated from the specimen in a manner known per se and then, washed with a pyrogen-free water, a pyrogen-free buffer solution or the like to remove substances other than the pyrogen.

In the present invention, the adsorbent thus washed can be directly subjected to the Limulus test as it is,
In general, the Limulus test is classified into synthetic substrate method and gelation method and the synthetic substrate method is further classified into colorimetry and fluorimetry according to a particular kind of the substrate. In the present invention, any of these methods can be employed. For example, the adsorbent wherein the pyrogen has been adsorbed is reacted with Limulus lysate at 25 to 40°C, usually, 37°C for 10 to 120 minutes, turbidity of the reaction solution due to gelation is measured with time, and then, the concentration of the pyrogen in the specimen can be determined based on a calibration curve which is made separately by using an authentic sample (e.g. LPS, etc.) according to the same operation as that described above. Alternatively, the adsorbent wherein the pyrogen has been adsorbed is reacted with Limulus lysate and a synthetic substrate having a chromophore or fluorophore under the same conditions. After completion of the reaction, the reaction mixture is subjected to colorimetry or fluorimetry and then the pyrogen content in the specimen is determined according to a calibration curve which is made separately according to the same manner as that described above. In the case of colorimetry, the determination can be carried out by using a synthetic substrate, for example, Boc-Leu-Gly-Arg-pNA (wherein Boc is t-butoxycarbonyl and pNA is p-nitroaniline) and measuring the absorbancy at the wavelength of 405 nm. In the case of fluorimetry, the determination can be carried out by using a synthetic substrate, for example, Boc-Leu-Gly-Arg-MCA (wherein Boc is the same as defined above and MCA is 7-amino-4-methylcumarin) and measuring the fluorescence at the excitation wavelength of 380 nm and the fluorescent wavelength of 460 nm.

The adsorbent used in the present invention can be produced, for example, according to the same manner as that described in GB-A-2092470.

According to the present invention, determination sensitivity of Limulus test can be extremely improved and the pyrogen content of a specimen even containing interfering substances which has hitherto been considered to be difficult to measure can also be determined. Further, even if Limulus test positive substances other than pyrogens are contained in the specimen, separation and determination of pyrogens can be carried out. Furthermore, it is possible to use a cheaper reagent instead of Limulus reagent. Thus, the method for determination of a pyrogen content of the present invention is suitable for applying to check during the production of medicines and the like.

The following Examples and Reference Examples further illustrate the present invention in detail.

### Example 1

### Study on calibration curve

To pyrogen-free water (3 ml) was added a predetermined amount of LPS (derived from E. coli O128: B12) to prepare an aqueous LPS solution (concentration of LPS: 0, 1, 2 or 3 pg/ml). To the solution was added the adsorbent prepared in Reference Example 1 hereinafter (30 mg, wet weight) and the mixture was stirred at 50 r.p.m. at room temperature for one hour. Then, it was centrifuged at 3000 r.p.m. for 5 minutes and the precipitated adsorbent was thoroughly washed with pyrogen-free water (3 ml). To the precipitate was added a substrate solution, i.e., an aqueous solution of 0.4 mM Boc-Leu-Gly-Arg-MCA (50 µl) and Limulus amebocyte lysate [prepared by dissolving 1 vial (for 0.2 ml, sensitivity: gelled at 0.1 ng/ml as FDA reference endotoxin EC-2 concentration) of Limulus single test Wako (manufactured by Wako Pure Chemical Industries, Ltd.) into 0.4 M Tris hydrochloride buffer (pH 8.0, 1.5 ml) containing 0.04 M magnesium chloride] (50 µl) and the mixture was reacted at 37°C for 30 minutes. After completion of the reaction, a 12.5% aqueous acetic acid solution (2.3 ml) was added to terminate the reaction and the mixture was centrifuged at 3000 r.p.m. for 5 minutes. Then, fluorimetry was carried out at 380 nm of excitation wavelength and 460 nm of fluorescent wavelength. The results are shown in Table 1.

When the data obtained were plotted on a graph wherein ordinates indicated the relative intensity (RI) in fluorimetry and abscissas indicated LPS concentration to obtain the calibration curve as shown in Fig. 1.

As seen from Fig. 1, the calibration curve shows good linear relationship even in the low LPS concentration region.

**Table 1**

| Run No. | LPS concentration (pg/ml) | RI |
|---|---|---|
| 1 | 0.0 | 13.6 |
| 2 | 0.0 | 17.1 |
| 3 | 1.0 | 54.0 |
| 4 | 1.0 | 59.0 |
| 5 | 2.0 | 99.0 |
| 6 | 2.0 | 107.0 |
| 7 | 3.0 | 148.0 |
| 8 | 3.0 | 152.0 |

### Example 2

### Reproducibility of data obtained by the method of the present invention

To pyrogen-free water (3 ml) was added a predetermined amount of LPS (derived from E. coli O128: B12) was stirred to obtain an aqueous LPS solution containing LPS of 2 pg/ml. According to the same manner as that described in Example 1, LPS content (relative intensity RI in fluorimetry) in the LPS solution or pyrogen-free water (3 ml, corresponding to LPS concentration of 0 pg/ml) was determined by using the adsorbent (30 mg) prepared in Reference Example 1 hereinafter. The results obtained by 4 runs per each solution are shown in Table 2.

**Table 2**

| | Amount of LPS (pg/ml) | |
|---|---|---|
| | 0 | 2 |
| RI | 8.9 | 73.2 |
| | 9.3 | 73.0 |
| | 7.8 | 73.7 |
| | 7.8 | 77.5 |

As is clear from Table 2, when the kind and the content of LPS are constant, the data obtained by the quantitative determination (relative intensity RI in fluorimetry) are also constant. Thus, reproducibility of the method of the present invention is high.

### Example 3

### Applicability of the method of the present invention to various pyrogens

Applicability of the method of the present invention to various pyrogens (LPS) was studied as follows.

### (1) Determination of LPS content according to the method of the present invention

To pyrogen-free water (3 ml) was added various LPS derived from microorganisms as described in Table 3 to obtain various aqueous LPS solutions having LPS concentrations of 1.25 to 6 pg/ml. According to the same manner as described in Example 1, LPS content in each solution was determined by using the adsorbent (30 mg) prepared in Reference Example 1 hereinafter. Endotoxin units (EU) of various LPS were determined based on LPS content of each specimen solution by using LPS derived from E. coli O128: B12 (10.4 EU/ng; EU represents Endotoxin units) as the endotoxin standard.

### (2) Determination of LPS content according to Limulus method using fluorescent synthetic substrate (control method).

To aqueous solutions of various LPS having a concentrations of 2.5 to 6 pg/ml (100 µl) was added the substrate solution used in Example 1 (50 µl) and Limulus amebocyte lysate used in Example 1 (50 µl), respectively, and the mixture was reacted at 37°C for 65 minutes. The reaction was terminated by addition of a 12.5 % aqueous acetic acid solution (3 ml). Then, according to the same manner as described in Example 1, LPS content was determined by measuring relative intensity in fluorimetry. According to the same manner as described above, endotoxin units of various LPS were determined by using the LPS derived from E. coli O128: B12 as the endotoxin standard. The results are shown in Table 3 below.

**Table 3**

| LPS | Method of present invention (EU/ng) | Control method (EU/ng) |
|---|---|---|
| LPS derived from E. coli O128: B12 | 10.4 | 10.4 |
| LPS derived from E. coli O111: B4 | 7.9 | 8.0 |
| LPS derived from E. coli O55: B5 | 11.6 | 12.1 |
| LPS derived from E. coli UKT-B | 24.9 | 22.7 |
| LPS derived from Klebsiella pneumoniae | 11.5 | 10.4 |

As is clear from Table 3, the method of the present invention is applicable to determination of content of various LPS as known Limulus method using a fluorescent synthetic substrate.

### Example 4

### Measurement of the concentration of the pyrogen in an aqueous phenylalanine solution

To a 2 % aqueous phenylalanine solution (30 ml) was added the adsorbent prepared in Reference Example 1 hereinafter (500 mg, wet weight) and the mixture was stirred at 50 r.p.m. at room temperature for 4 hours. Then, the adsorbent was removed to prepare a pyrogen-free aqueous phenylalanine solution.

To this aqueous phenylalanine solution was added LPS (derived from E. coli O128: B12) so that the concentration of LPS became 1 pg/ml to 2 pg/ml and the mixture was stirred to obtain LPS solution. To the solution (3 ml) was added the adsorbent prepared in Reference Example 1 hereinafter (30 mg, wet weight) and the mixture was stirred at room temperature for one hour. Then, it was centrifuged at 3000 r.p.m. for 5 minutes and the adsorbent precipitated was washed with pyrogen-free phosphate buffer (pH 7.0, µ= 0.02) (3 ml). To the precipitate was added an aqueous solution of 0.4 mM Boc-Leu-Gly-Arg-MCA (50 µl) as a substrate solution and Limulus amebocyte lysate [prepared by dissolving 1 vial (for 0.2 ml, sensitivity: gelled at 0.1 ng/ml as FDA reference endotoxin EC-2 concentration) of Limulus single test Wako (manufactured by Wako Pure Chemical Industries, Ltd.) into 0.4 M Tris hydrochloride buffer (pH 8.0, 1.5 ml) containing 0.04 M magnesium chloride] (50 µl) and the mixture was reacted at 37°C for 30 minutes. After completion of the reaction, a 12.5 % aqueous acetic acid solution was added to terminate the reaction and centrifuged at 3000 r.p.m. for 5 minutes. Then, fluorimetry was carried out at 380 nm of excitation wavelength and 460 nm of fluorescent wavelength. The concentration of the pyrogen was determined by the relative intensity based on the calibration curve. The results are shown in Table 4.

**Table 4**

| Amount of LPS (pg/ml) | RI | LPS content (pg/ml) |
|---|---|---|
| none | 20.7 | 0.01 |
| 1 | 83.3 | 0.99 |
| 2 | 149.0 | 2.02 |

Hitherto, the limit of determination of this type of LPS by Limulus test was the concentration of 1 pg/ml in a 0.5% aqueous phenylalanine solution, i.e., 200 pg per 1 g of the amino acid. On the other hand, as is clear from Table 4, LPS of 1 pg/ml in a 2% aqueous phenylalanine solution, i.e., 50 pg per 1 g of the amino acid can be determined according to the present invention. Thus, the sensitivity is extremely improved.

### Example 5

### Measurement of the concentration of the pyrogen in an aqueous solution of methionine

To a 5 % aqueous methionine solution (30 ml) was added the adsorbent prepared in Reference Example 1 hereinafter (500 mg, wet weight) and the mixture was stirred at 50 r.p.m. at room temperature for 4 hours. Then, the adsorbent was removed to prepare a pyrogen-free aqueous methionine solution.

According to the same manner as that described in Example 4, LPS was added to the resulting solution to determine the pyrogen content. The results are shown in Table 5.

**Table 5**

| Amount of LPS (pg/ml) | RI | LPS content (pg/ml) |
|---|---|---|
| none | 26.9 | 0.11 |
| 1 | 84.9 | 1.02 |
| 2 | 148.0 | 2.01 |

In the case of an aqueous methionine solution, hitherto, the limit of determination of LPS content was 100 pg per 1 g of the amino acid. However, as is clear from Table 5, LPS of 1 pg/ml in a 5% aqueous methionine solution, i.e., 20 pg per 1 g of the amino acid can be determined according to the present invention. Thus, the sensitivity is extremely improved.

### Example 6

### Measurement of the concentration of the pyrogen in an aqueous solution of cysteine hydrochloride

A 0.25 % aqueous cysteine hydrochloride solution hydrochloride was adjusted to pH 6.1 with sodium hydroxide and to the solution was added LPS to prepare aqueous cysteine hydrochloride solutions containing 1 pg/ml and 2 pg/ml of LPS, respectively.

According to the same manner as described in Example 4, LPS content was determined using the adsorbent prepared in Reference Example 1 hereinafter. Pyrogen-free water (3 ml) was used for washing the adsorbent. The results are shown in Table 6.

**Table 6**

| Amount of LPS (pg/ml) | RI | LPS content (pg/ml) |
|---|---|---|
| none | 16.3 | 0 |
| 1 | 69.8 | 1.04 |
| 2 | 118.0 | 2.05 |

In the case of an aqueous cysteine hydrochloride solution, hitherto, the limit of determination of LPS was 1000 to 2000 pg per 1 g of the amino acid. However, as is clear from Table 6, LPS of 1 pg/ml in a 0.25% cysteine hydrochloride solution, i.e., 400 pg per 1 g of the amino acid can be determined according to the present invention and sensitivity is extremely improved.

### Example 7

### Measurement of the concentration of the pyrogen in an aqueous solution of penicillin G

To a 1 % aqueous solution of penicillin G (30 ml) was added the adsorbent prepared in Reference Example 1 hereinafter (1 g, wet weight) and the mixture was stirred at 50 r.p.m. at room temperature for 4 hours. Then, the adsorbent was removed to give a pyrogen-free aqueous solution of penicillin G.

According to the same manner as described in Example 4, to the solution was added a predetermined amount of LPS and LPS content was determined using the adsorbent prepared in Reference Example 1 hereinafter. Pyrogen-free water (3 ml) was used for washing the adsorbent. The results are shown in Table 7.

**Table 7**

| Amount of LPS (pg/ml) | RI | LPS content (pg/ml) |
|---|---|---|
| none | 5.5 | 0.00 |
| 1 | 32.3 | 0.90 |
| 2 | 69.5 | 2.07 |

In the case of an aqueous solution of penicillin G, hitherto, the limit of determination of LPS was 200 pg per 1 g of penicillin G. However, as is clear from Table 7, LPS of 1 pg/ml in a 1% aqueous solution of penicillin G, that is, 100 pg per 1 g of penicillin G can be determined according to the present invention and sensitivity is extremely improved.

### Example 8

### Operation for determining the pyrogen content according to the present invention

To pyrogen-free water (3 ml) was added the predetermined amount of LPS (derived from E. coli O128: B12) and the mixture was stirred to prepare an aqueous solution of LPS (the concentration of LPS: 0, 10 and 20 pg/ml). To the solution was added the adsorbent prepared in Reference Example 1 hereinafter (100 mg, wet weight) and the mixture was stirred at 50 r.p.m. at room temperature for one hour. Then, it was centrifuged at 3000 r.p.m. for 5 minutes. To the adsorbent precipitated was added 0.2 M pyrogen-free Tris hydrochloride buffer (pH 8.0, 0.5 ml) containing 0.02 M magnesium chloride and the mixture was stirred and then allowed to stand for 30 minutes. After standing it was centrifuged and the concentration of LPS in the supernatant was determined according to Limulus method using a fluorescent synthetic substrate as follows.

The supernatant liquid was successively diluted with pyrogen-free water to give a sample solution. To the sample solution (100 µl) was added an solution of substrate (50 µl) and Limulus amebocyte lysate (50 µl) and the mixture was reacted at 37°C for 90 minutes. The reaction was terminated by addition of a 12.5 % aqueous acetic acid solution (2.3 ml) and then, according to the same manner as described in Example 1, relative intensity in fluorimetry was determined and LPS content was determined using LPS derived from E. coli O128: B12 as LPS standard. The results are shown in Table 8.

**Table 8**

| Run No. | LPS added (pg) | LPS measured (pg) |
|---|---|---|
| 1 | 0 | < 0.1 |
| 2 | 0 | < 0.1 |
| 3 | 30 | 26 |
| 4 | 30 | 36 |
| 5 | 60 | 41 |
| 6 | 60 | 62 |

As is clear from Table 8, LPS added was almost recovered to the supernatant eluted. Therefore, it is possible to measure the concentration of LPS by adsorbing it on the adsorbent and then eluting it.

### Example 9

### Study on calibration curve

To a pyrogen-free phosphate buffer (pH 7.0, µ=0.02, 3 ml) was added a predetermined amount of LPS (derived from E. coli O128: B12) to prepare an aqueous solution of LPS (the concentration of LPS: 0, 5, 10 or 15 pg/ml). To the solution was added the adsorbent prepared in Reference Example 2 hereinafter (30 mg, wet weight) and the mixture was stirred at 50 r.p.m. at room temperature for one hour. Then, it was centrifuged at 3000 r.p.m. for 5 minutes. To the precipitate was added an aqueous solution of 0.4 mM Boc-Leu-Gly-Arg-MCA (50 µl) as an solution of substrate and Limulus amebocyte lysate [prepared by dissolving 1 vial (for 0.2 ml, sensitivity: gelled at 0.1 ng/ml as FDA reference endotoxin EC-2 concentration) of Limulus single test Wako (manufactured by Wako Pure Chemical Industries, Ltd.) into 0.4 M Tris hydrochloride buffer (pH 8.0, 1.5 ml) containing 0.04 M magnesium chloride] (50 µl) and the mixture was reacted at 37°C for 20 minutes. After completion of the reaction, a 12.5 % aqueous acetic acid solution (2.3 ml) was added to terminate the reaction and centrifuged at 3000 r.p.m. for 5 minutes. Then, fluorimetry was carried out at 380 nm of excitation wavelength and 460 nm of fluorescent wavelength. The results are shown in Table 9.

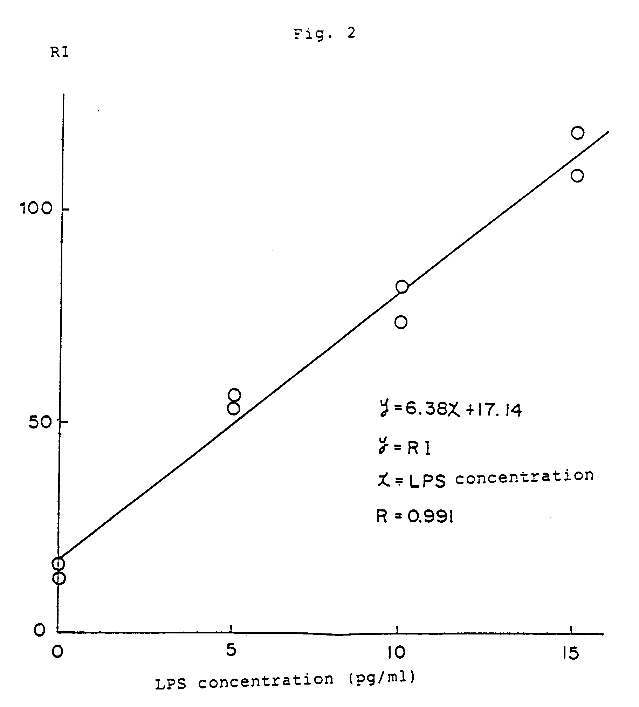

The resulting data were plotted on a graph wherein ordinates indicated a relative intensity (RI) in fluorimetry and abscissas indicated LPS concentration to obtain the calibration curve as shown in Fig. 2. The curve of Fig. 2 has a good linear relationship even in the low LPS concentration region.

**Table 9**

| Run No. | LPS concentration (pg/ml) | RI |
|---|---|---|
| 1 | 0 | 12.9 |
| 2 | 0 | 16.1 |
| 3 | 5 | 52.8 |
| 4 | 5 | 56.2 |
| 5 | 10 | 73.7 |
| 6 | 10 | 82.0 |
| 7 | 15 | 108 |
| 8 | 15 | 118 |

### Example 10

### Study on calibration curve

According to the same manner as described in Example 9, fluorimetry was carried out except that the adsorbent prepared in Reference Example 3 hereinafter was used in place of that prepared in Reference Example 2. The results are shown in Table 10.

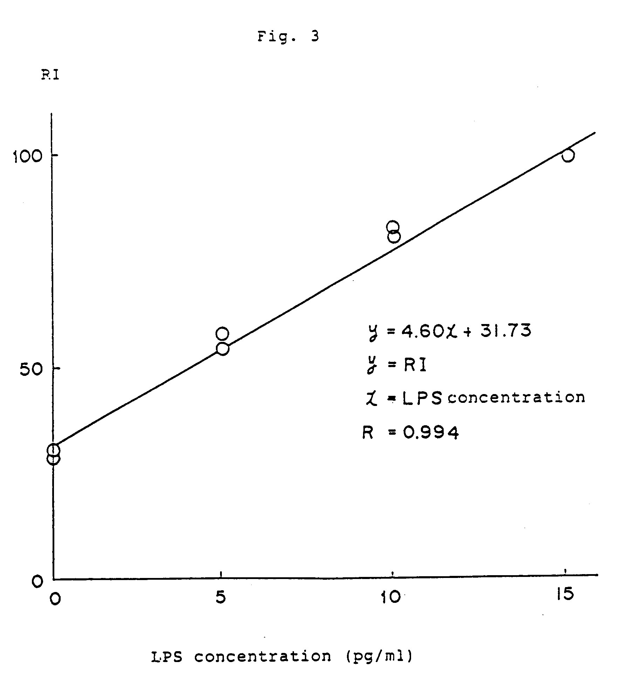

The resulting data were plotted on a graph wherein the ordinates indicated a relative strength (RI) in fluorimetry and the abscissas indicated LPS concentration to obtain a calibration curve as shown in Fig. 3. The curve of Fig. 3 shows a good linear relationship even in the low LPS concentration region.

**Table 10**

| Run No. | LPS concentration (pg/ml) | RI |
|---|---|---|
| 1 | 0 | 29.0 |
| 2 | 0 | 30.4 |
| 3 | 5 | 54.1 |
| 4 | 5 | 57.8 |
| 5 | 10 | 82.3 |
| 6 | 10 | 80.5 |
| 7 | 15 | 97.8 |
| 8 | 15 | 98.5 |

### Reference Example 1

(1) Sepharose CL-4B (trade name of an agarose derivative manufactured by Pharamacia Fine Chemicals, 30 g, wet weight) was suspended in a distilled water. 2 N Aqueous sodium hydroxide (200 ml) and epichlorohydrin (50 ml) were added and the mixture was stirred at 40°C for 2 hours. After completion of the reaction, the mixture was filtered and the residue was washed with a distilled water to obtain epichlorohydrin-activated Sepharose CL-4B. Epichlorohydrin-activated Sepharose CL-4B thus obtained was suspended in a 0.6% aqueous solution of hexamethylenediamine (1.4 liters) which had been warmed to 60°C and the suspension was stirred at 60°C for 2 hours. After completion of the reaction, the mixture was filtered and the residue was washed with a distilled water to give 3-(6-aminohexylamino)-2-hydroxy-propylated Sepharose CL-4B (370 g, wet weight). When the aminohexyl content of the resulting Sepharose was measured by titration, it was about 37.6 µmol/g (wet weight).
(2) 3-(6-Aminohexylamino)-2-hydroxy-propylated Sepharose CL-4B (370 g, wet weight) was suspended in 4 N aqueous sodium hydroxide (700 ml). Epichlorohydrin (700 ml) was added to the suspension at 65°C and the mixture was stirred. After the temperature of the mixture was reached 90°C, it was stirred for additional 8 minutes. After completion of the reaction, water was added to the mixture to cool to 50°C or lower. Then, the mixture was filtered, and the residue was washed with a distilled water to give epichlorohydrin-activated 3-(6-aminohexylamino)-2-hydroxy-propylated Sepharose CL-4B. This was suspended in an aqueous solution of 20% histidine hydrochloride hydrate (adjusting to pH 12 with an aqueous sodium hydroxide, 2.1 liters) which had been heated to 90°C and the suspension was stirred at 80 to 90°C for 30 minutes. After completion of the reaction, the mixture was filtered and the residue was thoroughly washed with distilled water. The residue was suspended in distilled water (1 liter) and subjected to autoclaving at 120°C for 20 minutes and then filtered. The residue was thoroughly washed in turn with 0.2 N aqueous hydrochloric acid, 0.2 N aqueous sodium hydroxide, 1.5 M aqueous sodium chloride and distilled water. Thus, water-insoluble adsorbent containing agarose as a carrier, histidine as a ligand and hexamethylenediamine as a spacer center (390 g, wet weight) was obtained. The content of histidine per 1 g (wet weight) of the adsorbent was 20.4 µmol.

### Reference Example 2

The same procedure as described in Reference Example 1 was repeated except that Cellulofine GCL-2000-m trade name of a cellulose derivative manufactured by Chisso Co., Ltd., 350 g) was used in place of Sepharose CL-4B. Thus, the water-insoluble adsorbent containing cellulose as a carrier, histidine as a ligand and hexamethylenediamine as a spacer center (340 g, wet weight) was obtained. The content of histidine per 1 g (wet weight) of the adsorbent was 58 µmol.

### Reference Example 3

3-(6-Aminohexylamino)-2-hydroxy-propylated Sepharose CL-4B prepared according to the same manner as that described in Reference Example 1 (18 g, wet weight) was suspended in 0.05 M phosphate buffer (pH 7.0, 45.6 ml). A 25 % aqueous solution of glutaraldehyde (19.2 ml) was added to the suspension and the mixture was stirred at room temperature for 2 hours. After completion of the reaction, the mixture was filtered and the residue was thoroughly washed with 0.1 M phosphate buffer (pH 7.0) to obtain glutaraldehyde-activated 3-(6-aminohexylamino)-2-hydroxy-propylated Sepharose CL-4B. This was suspended in 15 mM histamine-0.1 M phosphate buffer (pH 7.0, 59.6 ml) and the suspension was stirred at room temperature for 2 hours. After completion of the reaction, the mixture was filtered, and the residue was washed with a 1 M aqueous solution of sodium chloride (600 ml). The residue was suspended in 0.1 M phosphate buffer (pH 7.0, 30 ml). Sodium borohydride (0.3 g) was added to the suspension and the mixture was stirred at room temperature for an hour. After completion of the reaction, the mixture was filtered and the residue was thoroughly washed with 1 M aqueous sodium chloride solution and distilled water. Thus, water-insoluble adsorbent containing agarose as a carrier, histamine as a ligand and hexamethylenediamine as a spacer center (20.7 g, wet weight) was obtained. The content of histamine per 1 g (wet weight) of the adsorbent was 4.1 µmol.

## Claims

1. A method for determination of a pyrogen content which comprises bringing a specimen into contact with an adsorbent composed of a nitrogen-containing heterocyclic compound bonded to a water-insoluble carrier through a spacer, and without eluting the pyrogen adsorbed on the adsorbent, determining the content of the adsorbed pyrogen by means of Limulus method;
wherein said nitrogen-containing heterocyclic compound is compound (I) of formula
R-A-X (I)
wherein R is a nitrogen-containing heterocyclic group having an imidazole nucleus or a purine nucleus;
A is a single bond or an alkylene group;
X is a functional group;
and the alkylene group may be substituted with a carboxyl group; and said spacer being a compound of the formula:
NH₂(CH₂)ₙ NH₂,
NH₂(CH₂)ₙ COOH
NH₂(CH₂)ₙ OH
or
HOOC(CH₂)ₙ COOH
wherein n is an integer of 1 to 12.

2. The method according to claim 1, wherein in compound (I) A is a single bond or an alkylene group having 1 to 12 carbon atoms which may be substituted with a carboxyl group; and X is amino, hydroxy or carboxyl.

3. The method according to claim 1, wherein in compound (I) A is a single bond, ethylene or ethylene substituted with a carboxyl group; and X is an amino group.

4. The method according to claim 1, wherein the nitrogen-containing heterocyclic compound (I) is selected from histidine, histamine and adenine.

5. The method according to claim 1, 2, 3 or 4, wherein the water-insoluble carrier is a water-insoluble carrier having a hydroxy group, amino group or a halogen atom in the molecule thereof.

6. The method according to claim 5, wherein the adsorbent is compound (I) bonded to the water-insoluble carrier through an alkylenediamine as the spacer by using, at least, either one of the monoepoxide and an aliphatic aldehyde.

7. The method according to claim 6, wherein the adsorbent is compound (I) is bonded to the water-insoluble carrier through the spacer by using epichlorohydrin as the monoepoxide.

8. The method according to claims 6 or 7, wherein the water-insoluble carrier is a water insoluble polysaccharide.

9. The method according to claim 8, wherein the water-insoluble carrier is agarose or cellulose.

10. A method according to claim 1, wherein the specimen is a water-containing solution or an aqueous solution.

11. A method according to claim 1, wherein the adsorbent is used in an amount of 3 to 100 mg per 1 ml of a specimen and the adsorption is carried out under conditions of a temperature of 4 to 40°C, pH of 4 to 8 and an ionic strength of not more than 0.1.

## Patentansprüche

1. Verfahren zur Bestimmung des Pyrogengehalts, wobei man ein Spezimen in Kontakt mit einem Adsorbens bringt, das aus einer stickstoffhaltigen heterozyklischen Verbindung zusammengesetzt ist, die an einen wasserunlöslichen Träger über einen Spacer gebunden ist, und wobei man ohne Eluierung des am Adsorbens adsorbierten Pyrogens den Pyrogengehalt des adsorbierten Pyrogens mit der Limulus-Methode bestimmt,
worin die genannte stickstoffhaltige heterozyklische Verbindung Verbindung (I) der Formel ist:
R-A-X (I)
worin R eine stickstoffhaltige heterozyklische Gruppe mit einem Imidazol- oder Purin-Kern,
A eine Einfachbindung oder eine Alkylengruppe, und
X eine funktionelle Gruppe sind,
wobei die Alkylengruppe mit einer Carboxylgruppe substituiert sein kann, und worin der genannte Spacer eine Verbindung der Formel ist:
NH₂(CH₂)ₙNH₂,
NH₂(CH₂)ₙCOOH,
NH₂(CH₂)ₙOH
oder
HOOC(CH₂)ₙCOOH,
worin n eine ganze Zahl von 1 bis 12 ist.

2. Verfahren gemäß Anspruch 1, worin in Verbindung (I) A eine Einfachbindung oder eine Alkylengruppe mit 1 bis 12 Kohlenstoffatomen darstellt, die mit einer Carboxylgruppe substituiert sein kann, und worin X eine Amino-, Hydroxy- oder Carboxylgruppe ist.

3. Verfahren gemäß Anspruch 1, worin in Verbindung (I) A eine Einfachbindung, Ethylen- oder mit einer Carboxylgruppe substituierte Ethylengruppe und X eine Aminogruppe darstellen.

4. Verfahren gemäß Anspruch 1, worin die stickstoffhaltige heterozyklische Verbindung (I) aus Histidin, Histamin und Adenin ausgewält ist.

5. Verfahren gemäß Anspruch 1, 2, 3 oder 4, worin der wasserunlösliche Träger ein wasserunlöslicher Träger mit einer Hydroxygruppe, Aminogruppe oder einem Halogenatom im Molekül davon ist.

6. Verfahren gemäß Anspruch 5, worin das Adsorbens Verbindung (I) ist, die an den wasserunlöslichen Träger über ein Alkylendiamin als Spacer gebunden ist, durch Verwendung von mindestens einem Monoepoxid und einem aliphatischen Aldehyd.

7. Verfahren gemäß Anspruch 6, worin das Adsorbens Verbindung (I) ist, die an den wasserunlöslichen Träger über den Spacer durch Verwendung von Epichlorhydrin als Monoepoxid gebunden ist.

8. Verfahren gemäß Anspruch 6 oder 7, worin der wasserunlösliche Träger ein wasserunlösliches Polysaccharid ist.

9. Verfahren gemäß Anspruch 8, worin der wasserunlösliche Träger Agarose oder Cellulose ist.

10. Verfahren gemäß Anspruch 1,
worin das Spezimen eine wasserhaltige Lösung oder eine wässrige Lösung ist.

11. Verfahren gemäß Anspruch 1,
worin das Adsorbens in einer Menge von 3 bis 100 mg pro 1 ml eines Spezimen verwendet und die Adsorption unter Bedingungen einer Temperatur von 4 bis 40°C, einem pH von 4 bis 8 und einer Ionenstärke von nicht mehr als 0,1 durchgeführt werden.

## Revendications

1. Méthode de détermination d'une teneur en pyrogène qui comprend la mise en contact d'un échantillon avec un adsorbant constitué par un composé hétérocyclique azoté lié à un support insoluble dans l'eau par l'intermédiaire d'un espaceur, et, sans élution du pyrogène adsorbé sur l'adsorbant, la détermination de la teneur en pyrogène adsorbé par la méthode du Limulus; dans laquelle ledit composé hétérocyclique azoté est un composé (I) de formule
R-A-X (I)
dans laquelle R est un groupe hétérocyclique azoté ayant un noyau d'imidazole ou un noyau de purine;
A est une liaison simple ou un groupe alkylène;
X est un groupe fonctionnel;
et le groupe alkylène peut être substitué par un groupe carboxyle; et ledit espaceur est un composé de formule:
NH₂(CH₂)ₙNH₂,
NH₂(CH₂)ₙCOOH,
NH₂(CH₂)ₙOH
ou
HOOC(CH₂)ₙCOOH
où n est un entier de 1 à 12.

2. Méthode selon la revendication 1, dans laquelle, dans le composé (I), A est une liaison simple ou un groupe alkylène ayant 1 à 12 atomes de carbone qui peut être substitué par un groupe carboxyle; et X est un groupe amino, hydroxy ou carboxyle.

3. Méthode selon la revendication 1, dans laquelle, dans le composé (I), A est une liaison simple, un éthylène ou un éthylène substitué par un groupe carboxyle; et X est un groupe amino.

4. Méthode selon la revendication 1, dans laquelle le composé hétérocyclique azoté (I) est choisi parmi l'histidine, l'histamine et l'adénine.

5. Méthode selon la revendication 1, 2, 3 ou 4, dans laquelle le support insoluble dans l'eau est un support insoluble dans l'eau ayant dans sa molécule un groupe hydroxy, un groupe amino ou un atome d'halogène.

6. Méthode selon la revendication 5, dans laquelle l'adsorbant est un composé (I) lié au support insoluble dans l'eau par l'intermédiaire d'une alkylènediamine comme espaceur à l'aide d'au moins l'un ou l'autre d'un monoépoxyde et d'un aldéhyde aliphatique.

7. Méthode selon la revendication 6, dans laquelle l'adsorbant est le composé (I) lié au support insoluble dans l'eau par l'intermédiaire de l'espaceur à l'aide d'épichlorhydrine en tant que monoépoxyde.

8. Méthode selon les revendications 6 ou 7, dans laquelle le support insoluble dans l'eau est un polysaccharide insoluble dans l'eau.

9. Méthode selon la revendication 8, dans laquelle le support insoluble dans l'eau est de l'agarose ou de la cellulose.

10. Méthode selon la revendication 1, dans laquelle l'échantillon est une solution contenant de l'eau ou une solution aqueuse.

11. Méthode selon la revendication 1, dans laquelle on utilise l'adsorbant en une quantité de 3 à 100 mg par ml d'échantillon et on effectue l'adsorption dans les conditions d'une température de 4 à 40°C, d'un pH de 4 à 8 et d'une force ionique inférieure ou égale à 0,1.
